# EUROPÄISCHE PATENTSCHRIFT

(11) **EP 1 811 990 B1**
(45) Veröffentlichungstag und Bekanntmachung des Hinweises auf die Patenterteilung: **21.11.2018**
(21) Anmeldenummer: 05802055.3
(22) Anmeldetag: 27.10.2005
(51) Int. Cl.: A61K 31/365, A61P 17/00

(54) **MITTEL GEGEN DEMODIKOSE**
ANTI-DEMODICOSIS AGENT
AGENT POUR LUTTER CONTRE LA GALE DEMODECTIQUE

(30) Priorität: 09.11.2004 DE 102004053964
(43) Veröffentlichungstag der Anmeldung: 01.08.2007
(73) Patentinhaber: Bayer Intellectual Property GmbH, 40789 Monheim (DE)
(72) Erfinder: HEINE, Josef, 42799 Leichlingen (DE); KRIEGER, Klemens, 51789 Lindlar (DE)
(74) Vertreter: BIP Patents
(86) Internationale Anmeldenummer: PCT/EP2005/011484
(87) Internationale Veröffentlichungsnummer: WO 2006/050816

(56) Entgegenhaltungen:
- EP-A- 0 421 568
- WO-A-92/08455
- DE-A1- 10 117 676
- US-A- 5 952 372
- DATABASE BIOSIS [Online] BIOSCIENCES INFORMATION SERVICE, PHILADELPHIA, PA, US; 2003, STRABEL D ET AL: "Treatment with avermectins in two goats with demodicosis." XP002364797 Database accession no. PREV200400135858 & SCHWEIZER ARCHIV FUER TIERHEILKUNDE, Bd. 145, Nr. 12, 2003, Seiten 585-588, ISSN: 0036-7281
- MURTHY T V R ET AL: "THERAPEUTIC TRIALS OF DEMODECTIC MANGE IN DOGS WITH AMITRAZ AND IVERMECTIN" INDIAN VETERINARY MEDICAL JOURNAL, UP VETERINARY ASSOCIATION, LUCKNOW, IN, Bd. 17, Nr. 1, März 1993 (1993-03), Seiten 28-29, XP008051926 ISSN: 0250-5266
- MUELLER RALF S: "Treatment protocols for demodicosis: an evidence-based review." VETERINARY DERMATOLOGY. APR 2004, Bd. 15, Nr. 2, April 2004 (2004-04), Seiten 75-89, XP002364791 ISSN: 0959-4493 in der Anmeldung erwähnt
- HEINE J ET AL: "Evaluation of the efficacy and safety of imidacloprid 10% plus moxidectin 2.5% spot-on in the treatment of generalized demodicosis in dogs: Results of a European field study" PARASITOLOGY RESEARCH 2005 GERMANY, Bd. 97, Nr. SUPPL. 1, 2005, Seiten S89-S96, XP002364792 ISSN: 0932-0113
- PARADIS M: "New approaches to the treatment of canine demodicosis." THE VETERINARY CLINICS OF NORTH AMERICA. SMALL ANIMAL PRACTICE. NOV 1999, Bd. 29, Nr. 6, November 1999 (1999-11), Seiten 1425-1436, XP008059101 ISSN: 0195-5616 in der Anmeldung erwähnt
- R Wagner ET AL: "Field efficacy of moxidectin in dogs and rabbits naturally infested with Sarcoptes spp., Demodex spp. and Psoroptes spp. mites", Veterinary Parasitology, vol. 93, no. 2, 1 November 2000 (2000-11-01), pages 149-158, XP055117893, ISSN: 0304-4017, DOI: 10.1016/S0304-4017(00)00357-5

## Beschreibung

Die vorliegende Erfindung betrifft die Verwendung von Arzneimitteln zur äußerlichen Anwendung zur Behandlung von Demodikose, insbesondere beim Hund, wobei die Arzneimittel Moxidectin und ein Neonicotinoid enthalten.
Makrocyclische Lactone sind vor allem in der Veterinärmedizin bekannt als Mittel, die sowohl hervorragende endoparasitizide Wirkung sowie in gewissen Grenzen auch ektoparasitizide Wirkung aufweisen. So ist z.B. eine Wirkung gegen ektoparasitäre Arthropoden bekannt.
Bei der Demodikose, insbesondere des Hundes, wird zwischen einer juvenilen in der Regel selbst heilenden lokalen Erkrankung (engl.: "localized demodicosis") und einer beim erwachsenen Tier auftretenden generalisierten Erkrankung (engl.: "generalized demodicosis") unterschieden. Die generalisierte Demodikose stellt eine schwere klinische Erkrankung dar, die äußerst schwierig zu therapieren ist. Zur Therapie der Demodikose wurden zunächst Waschungen mit acariziden Mitteln, z.B. Ronnel (O,O-dimethyl O-(2,4,5-trichlorphenyl) phosphorothioat) eingesetzt, die jedoch wegen eines hohen Vergiftungsrisikos bei Hund und Anwender abzulehnen sind. Modernere Mittel sind Waschungen mit Amitraz ((N'-(2,4-dimethylphenyl)-N[[(2,4-dimethylphenyl)imino]methyl]-methanimidamide) in 1-2 wöchigem Abstand. Ebenfalls erfogreich angewendet werden makrozyklische Laktone, die in täglichen bis wöchentlichen oder 2 wöchentlichen Abstand oral oder per injectionem verabreicht werden. Diese Behandlungsprogramme sind teuer für den Tierhalter und unbequem für Hund und Halter, der Erfolg ist nicht sicher. Die Spot-on Behandlung 3mal pro Woche mit Ivermectin wurde im Stand der Technik als nicht ausreichend wirksam beschrieben. Der Stand der Technik bezüglich der Demodikose-Behandlung ist dargestellt in: R. S. Mueller, Veterinary Dermatology, 15 (2004) 75-89; M. Paradis, Veterinary Clinics of North America: Small Animal Practice, 29(6) (1999) 1425-1436.
Überraschenderweise wurde nun gefunden, dass auch bei äußerlicher Anwendung des makrocyclischen Lactons Moxidectin eine gute Wirkung gegen Demodikose erreicht werden kann. Die Erfindung betrifft die Verwendung von Moxidectin zur Herstellung von Arzneimitteln zur äußerlichen Anwendung in Form einer pour-on oder spot-on Formulierung zur Behandlung von Demodikose, wobei die Formulierung 0,02 bis 8 % (m/m) Moxidectin sowie 1 bis 20 % (m/m) Neonicotinoid enthält und in einer Menge von 0,1 bis 20 ml auf einen kleinen Teil der Körperoberfläche des zu behandelnden Tieres aufgebracht wird. Makrocyclische Lactone sind insbesondere Avermectine, 22,23-Dihydroavermectine B₁ (Ivermectine) oder Milbemycine.

Avermectine wurden aus dem Mikroorganismus Streptomyces avermitilis als mikrobielle Metabolite isoliert (US-Pat. 4 310 519) und können im wesentlichen als Gemisch, bestehend aus den acht Komponenten A₁ₐ, A_{1b}, A₂ₐ A_{2b}, B₁ₐ, B_{1b}, B₂ₐ und B_{2b}, auftreten (I. Putter et al. Experentia 37 (1981) S. 963, Birkhäuser Verlag (Schweiz)). Daneben besitzen auch die synthetischen Derivate, insbesondere das 22,23-Dihydroavermectin B₁ (Ivermectin), Interesse (US-Pat. 4 199 569). Milbemycin B-41 D konnte ebenso fermentativ aus Streptomyces hygroscopicus isoliert werden (vgl. "Milbemycin: Discovery and Development" I. Junya et al. Annu. Rep. Sankyo Res. Lab. 45 (1993), S. 1-98; JP-Pat. 8 378 549; GB 1 390 336).

Der Einsatz von Avermectinen, 22,23 Dihydroavermectinen B₁ (Ivermectinen) und Milbemycinen aus der Klasse der makrocyclischen Lactone als Endoparasitizide ist lange bekannt und Gegenstand zahlreicher Patentanmeldungen sowie Übersichtsartikel (z. B. Biologische Wirkungen in: "Ivermectin and Abamectin" W. C. Campbell, Ed., Springer Verlag, New York, N. Y., 1989; "Avermectins and Milbemycins Part II" H. G. Davies et al. Chem. Soc. Rev. 20 (1991) S. 271-339; Chemische Modifikationen in: G. Lukacs et al. (Eds.), Springer-Verlag, New York, (1990), Chapter 3; Cydectin ™ [Moxidectin und Derivate]: G. T. Carter et al. J. Chem. Soc. Chem. Commun. (1987), S. 402-404); EP 423 445-A1). Der Einsatz von Doramectin (Pfizer) als Endoparasitizid ist ebenso bekannt (vgl. "Doramectin - a potent novel endectozide" A. C. Goudie et al. Vet. Parasitol. 49 (1993), S. 5-15).

Bei den Avermectinen handelt es sich um Stoffe oder Stoffgemische von makroliden Lactonen der allgemeinen Formel (I) 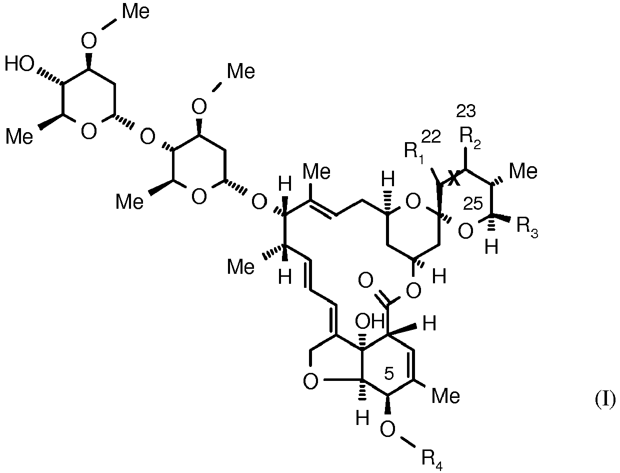 in welcher
die Reste R¹ bis R⁴ die in der nachfolgenden Tabelle 1 angegebene Bedeutung haben und X für eine Einfach- oder Doppelbindung zwischen der C₂₂- und C₂₃-Position (-C₂₂R¹-X-C₂₃R²-) stehen kann.

Im Falle einer Doppelbindung befinden sich keine Substituenten (R¹, R²) an der C₂₂- und C₂₃-Position.

**Tabelle 1**

| **Makrocyclisches Lacton** | **-C₂₂R¹-X-C₂₃R²-** | **R³** | **R⁴** |
|---|---|---|---|
| Avermectin A₁ₐ | -CH=CH- | -sec- Bu | -Me |
| Avermectin A_{1b} | -CH=CH- | -iso-Pr | -Me |
| Avermectin A₂ₐ | -CH₂-CHOH- | -sec- Bu | -Me |
| Avermectin A_{2b} | -CH₂-CHOH- | -iso-Pr | -Me |
| Avermectin B₁ₐ | -CH=CH- | -sec- Bu | -H |
| Avermectin B_{1b} | -CH=CH- | -iso-Pr | -H |
| Avermectin B₂ₐ | -CH₂-CHOH- | -sec- Bu | -H |
| Avermectin B_{2b} | -CH₂-CHOH- | -iso-Pr | -H |
| 22,23-Dihydroavermectin B₁ₐ | -CH₂-CH₂- | -sec- Bu | -H |
| 22,23-Dihydroavermectin B_{1b} | -CH₂-CH₂- | -iso-Pr | -H |
| Doramectin | -CH=CH- | -Chx | -H |

| | | | |
|---|---|---|---|
| 22,23-Dihydroavermectin B₁ steht für Ivermectin B₁; sec-Bu = sekundär Butyl; iso-Pr = Isopropyl; Chx = Cyclohexyl; -Me = Methyl | | | |

Die Avermectine und 22,23-Dihydroavermectine B₁ (Ivermectine) der allgemeinen Formel (I) werden in der Regel als Gemische eingesetzt. Von besonderem Interesse ist hierbei das Produkt Abamectin, das im wesentlichen die Avermectine B₁ enthält, und deren Hydrierungsprodukte die 22,23-Dihydroavermectine B₁ (Ivermectin).

Die mit "b" bezeichneten Verbindungen der makrocyclischen Lactone, die in der C₂₅-Position einen iso-Propylrest besitzen, müssen nicht notwendiger Weise von den "a" Verbindungen, welche eine sec-Butylgruppe in der C₂₅-Position haben, getrennt werden. Es wird generell das Gemisch beider Substanzen, bestehend aus > 80 % m/m sec-Butylderivat (B₁ₐ) und < 20 % m/m iso-Propylderivat (B_{1b}) isoliert, und kann verwendet werden. Zudem können bei den Stereoisomeren die Substituenten in der C₁₃- und C₂₃-Position sowohl α- als auch ß-ständig am Ringsystem angeordnet sein, d. h. sich oberhalb oder unterhalb der Molekülebene befinden. In jedem Fall werden alle Stereoisomeren berücksichtigt. Die 4:1-Mischung von Avermectin B₁ₐ und Avermectin B_{1b} wird in der Literatur als Abamectin bezeichnet.

Weiterhin leitet sich das semisynthetische makrocyclische Lacton Selamectin (5-Hydroxyimino-25-Cyclohexyl-Avermectin B₁-Monosaccharid) von den Avermectinen ab:

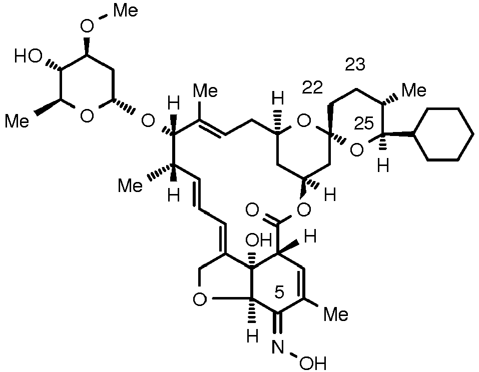

Ebenfalls von den Avermectinen leitet sich Eprinomectin ((4"*R*)-4"-(Acetylamino)-4"-deoxyavermectin B₁) ab; unter dieser Bezeichnung versteht man ein Mischung von 90% oder mehr der Komponente B₁ₐ und 10 % oder weniger der Komponente B_{1b}: 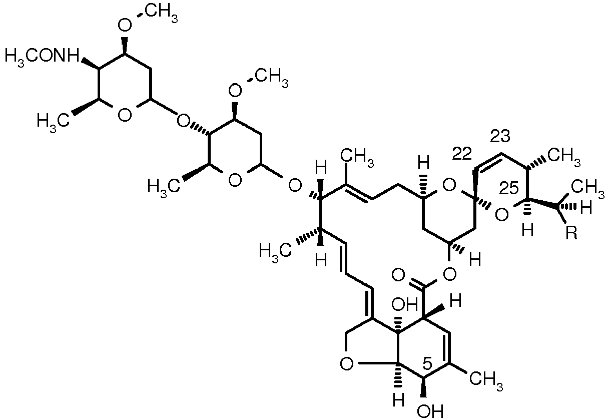
Komponente B₁ₐ: R = C₂H₅
Komponente B_{1b}: R = CH₃

Die Milbemycine haben die gleiche makrolide Ringstruktur wie Avermectine oder 22,23-Dihydroavermectine B₁ (Ivermectine), tragen aber keinen Substituenten (d.h. fehlendes Oleandrose Disaccharidfragment) in Position 13 (R⁵ = Wasserstoff).

Beispielhaft seien als Milbemycine aus der Klasse der macrocyclischen Lactone die Verbindungen mit der allgemeinen Formel (II) genannt 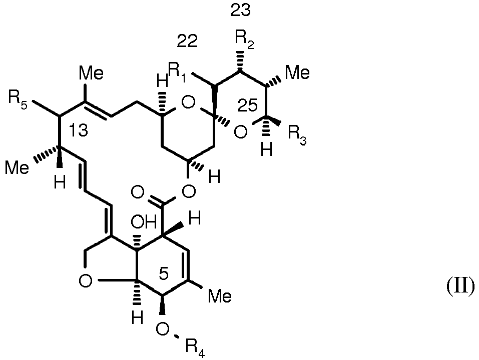 in welcher
die Reste R¹ bis R⁵ die in der nachfolgenden Tabelle 2 angegebene Bedeutung haben:

**Tabelle 2**

| **Makrocyclisches Lacton** | **R¹** | **R²** | **R³** | **R⁴** | **R⁵** |
|---|---|---|---|---|---|
| Milbemycin B41 D | -H | -H | -iso-Pr | -H | -H |
| Nemadectin | -H | -OH | 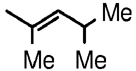 | -H | -H |
| Moxidectin | -H | =N-O-Me | 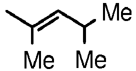 | -H | -H |

| | | | | | |
|---|---|---|---|---|---|
| iso-Pr = Isopropyl | | | | | |

Im Zusammenhang mit den Milbemycinen sei auch das Milbemycin-Oxim genannt, das in der Regel als Mischung von 80% Milbemycin A₄ 5-Oxim und 20 % Milbemycin A₃ 5-Oxim eingesetzt wird: 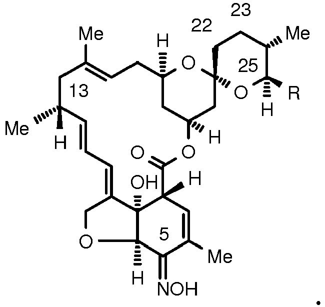
Milbemycin A₄ Oxim: R = -CH₂CH₃
Milbemycin A₄ Oxim: R = -CH₃

Soweit anwendbar werden im Sinne der Erfindung unter den Wirkstoffen auch deren pharmazeutisch annehmbare Salze, Hydrate verstanden.

Die Demodikose ist eine spezielle Form der auch als Räude bezeichneten Krankheit ("Demodex-Räude") und wird durch die Haarbalgmilben *Demodex spp*., insbesondere z.B. *Demodex canis*, hervorgerufen.

Die Demodikose kann bei verschiedenen Haus- und Nutztieren, wie z. B. bei Rindern oder Katzen auftreten, ist aber vor allem bei Hunden von besonderer Bedeutung. Erfindungsgemäß ist daher die Behandlung von Hunden bevorzugt.

Die Anwendung kann sowohl prophylaktisch als auch therapeutisch erfolgen.

Es hat sich gezeigt, dass unerwarteterweise auch bei äußerlicher Anwendung des Moxidectins eine gute und lang anhaltende Wirkung gegen die *Demodikose des Hundes* erreicht werden kann.

Die äußerliche Anwendung geschieht in Form des Aufgießens (pour-on and spot-on) eines kleinen Volumens, beispielsweise von 1-10 ml, auf einen Teil der Körperoberfläche des zu behandelnden Tieres. Besonders überraschend war dabei, dass gerade bei äußerlicher Applikation vergleichsweise kleiner Volumina eine gute und lang anhaltende Wirkung erzielt werden kann; die erfindungsgemäße Anwendung ist daher einfacher und anwenderfreundlicher als bisher bekannte Behandlungen der Demodikose.

Geeignete Zubereitungen sind:
Lösungen, beispielsweise Lösungen zum Gebrauch auf der Haut oder in Körperhöhlen, Aufgußformulierungen, Gele;
Emulsionen und Suspensionen, halbfeste Zubereitungen.

Lösungen zum Gebrauch auf der Haut werden aufgeträufelt, aufgestrichen, eingerieben, aufgespritzt, aufgesprüht. Diese Lösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden; auf steriles Arbeiten kann dabei verzichtet werden.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die pharmazeutisch geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt, indem Lösungen, die wie oben beschrieben hergestellt worden sind, mit soviel Verdickungsmittel versetzt werden, dass eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole wie Ethanol, Isopropanol, 2-Hexyldecanol, Octyldodecanol und Tetrahydrofurfurylalkohol, Glykole wie Glycerol, Propylenglykol, Polyethylenglykole, Polypropylenglykole, aromatisch substituierte Alkohole wie Benzylalkohol, Phenylethanol, Phenoxyethanol, Ester wie Essigester, Butylacetat, Benzylbenzoat, Dibutyladipat, Dicaprylylcarbonat, Diethylhexylcarbonat, Propylencarbonat, Ether wie Dicaprylylether, Alkylenglykolalkylether wie Dipropylenglycolmonomethylether, Diethylenglykolmonoethylether, Ketone wie Aceton, Methylethylketon, Methylisobutylketon, aromatische und/oder aliphatische Kohlenwasserstoffe, pflanzliche oder synthetische fette Öle wie Erdnussöl, Olivenöl, Rapsöl, Sesamöl, Sojaöl, Sonnenblumenöl, Glycerylricinoleat, Mittelkettige Triglyceride, Propylenglykoldicaprylat/dicaprat, Propylenglykoldipelargonat und Propylenglykollaurat; andere Fettsäureester wie 2-Octyldodecylmyristat, Cetearylisononanoat, Cetearyloctanoat, Cetylethylhexanoat, Cococaprylat/caprat, Decylcocoat, Decyloleat, Ethyloleat, Isocetylpalmitat, Isopropylmyristat, Isopropylpalmitat, Isostearylisostearat, Octylpalmitat, Octylstearat, Oleylerucat; Silikonöle wie Cethyldimethicon, Dimethicon und Simethicon; Dimethylformamid, Dimethylacetamid, Glycerolformal, Glycofurol, 2-Pyrrolidon, N-Methylpyrrolidon, 2-Dimethyl-4-hydroxy-methylen-1,3-dioxolan, Dioctylcyclohexan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Isopropylpalmitat, Dipropylenglykolpelargonat, Silikonöle, Fettsäureester, Triglyceride, Fettalkohole.

Antioxidantien sind Sulfite oder Metabisulfite wie Kaliummetabisulfat, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-triglycerid, Triglyceridgemisch mit Pflanzenfettsäure der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettelänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Ethanol, Isopropanol, Propylenglykol, Glycerol, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Stabilisatoren, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen unterscheiden sich von den oben beschriebenen Suspensionen und Emulsionen nur durch ihre höhere Viskosität.

Das Moxidectin kann auch in Kombination mit Synergisten oder mit weiteren Wirkstoffen vorliegen. Erfindungsgemäß wird es in Kombination mit Neonicotinoiden (Insektiziden aus der Gruppe der Agonisten der nicotinergen Acetylcholinrezeptoren von Insekten) eingesetzt. Mit solchen Kombinationen lassen sich neben der Demodikose auch ektoparasitäre Insekten und Endoparasiten bekämpfen.

Unter Neonicotinoiden sollen insbesondere Verbindungen der Formel (I) verstanden werden: 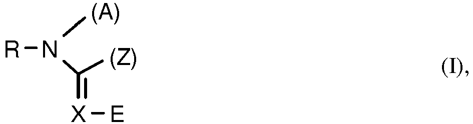 in welcher
- R: für Wasserstoff, gegebenenfalls substituierte Reste der Gruppe Acyl, Alkyl, Aryl, Aralkyl, Heteroaryl, Heteroarylalkyl oder Heterocyclylalkyl steht;
- A: für eine monofunktionelle Gruppe aus der Reihe Wasserstoff, Acyl, Alkyl, Aryl steht oder für eine bifunktionelle Gruppe steht, die mit dem Rest Z verknüpft ist;
- E: für einen elektronenziehenden Rest steht;
- X: für die Reste -CH= oder =N- steht, wobei der Rest -CH= anstelle eines H-Atoms mit dem Rest Z verknüpft sein kann;
- Z: für eine monofunktionelle Gruppe aus der Reihe Alkyl, -O-R, -S-R, 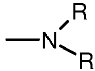 steht,
wobei
R für gleiche oder verschiedene Reste steht und die oben angegebene Bedeutung hat,
oder Z für eine bifunktionelle Gruppe steht, die mit dem Rest A oder dem Rest X verknüpft ist.

Besonders bevorzugt sind Verbindungen der Formel (I), in welcher die Reste folgende Bedeutung haben:
- R: steht für Wasserstoff sowie für gegebenenfalls substituierte Reste aus der Reihe Acyl, Alkyl, Aryl, Aralkyl, Heteroaryl, Heteroarylalkyl, Heterocyclylalkyl.
Als Acylreste seien genannt Formyl, (C₁₋₈-Alkyl)-carbonyl, (C₆₋₁₀-Aryl)-carbonyl, (C₁₋₈-Alkyl)-sulfonyl, (C₆₋₁₀-Aryl)-sulfonyl, (C₁₋₈-Alkyl)-(C₆₋₁₀-Aryl)-phosphoryl, die ihrerseits substituiert sein können. Als Alkyl seien genannt C₁₋₁₀-Alkyl, insbesondere C₁₋₄-Alkyl, im einzelnen Methyl, Ethyl, i-Propyl, sec.- oder t.-Butyl, die ihrerseits substituiert sein können.
Aryl ist insbesondere C₆₋₁₀-Aryl, als Beispiele seien genannt Phenyl, Naphthyl, insbesondere Phenyl.
Aralkyl ist insbesondere (C₆₋₁₀-Aryl)-(C₁₋₄-Alkyl), als Beispiele seien genannt Phenylmethyl, Phenethyl.
Als Heteroaryl seien genannt Heteroaryl mit bis zu 10 Ringatomen und N, O, S insbesondere N als Heteroatomen. Im einzelnen seien genannt Thienyl, Furyl, Thiazolyl, Imidazolyl, Pyridyl, Benzthiazolyl.
Heteroarylalkyl ist insbesondere Heteroaryl-(C₁₋₄-Alkyl), wobei Heteroaryl wie vorstehend definiert ist. Als Beispiele seien genannt Heteroarylmethyl, Heteroarylethyl mit bis zu 6 Ringatomen und N, O, S, insbesondere N als Heteroatomen.
Heterocyclyl ist insbesondere ein ungesättiger aber nicht aromatischer oder gesättigter Heterocyclus mit bis zu 6 Ringatomen, enthaltend bis zu 3 Heteroatome ausgewählt aus N, O, S, zum Beispiel Tetrahydrofuryl.
Heterocyclylalkyl ist insbesondere Heterocyclyl-C₁₋₂-Alkyl, z.B.: Tetrahydrofuranylmethyl und Tetrahydrofuranylethyl.
Als Substituenten seien beispielhaft und vorzugsweise aufgeführt:
Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo (-SO₃H); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.
- A: steht besonders bevorzugt für Wasserstoff sowie für gegebenenfalls substituierte Reste aus der Reihe Acyl, Alkyl, Aryl, die bevorzugt die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O, S unterbrochen sein können.
- A und Z: können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder. Als Beispiele für den heterocyclischen Ring seien Pyrrolidin, Piperidin, Piperazin, Hexamethylenimin, Hexahydro-1,3,5-triazin, Morpholin und Oxadiazin genannt, die gegebenenfalls bevorzugt durch Methyl substituiert sein können.
- E: steht für einen elektronentziehenden Rest, wobei insbesondere NO₂, CN, Halogenalkylcarbonyl wie Halogen-C₁₋₄-alkylcarbonyl mit 1 bis 9 Halogenatomen, insbesondere COCF₃, sowie C₁₋₄-Alkylsulfonyl und Halogen-C₁₋₄-alkylsulfonyl mit 1 bis 9 Halogenatomen, insbesondere SO₂CF₃, genannt seien.
- X: steht für -CH= oder -N=
- Z: steht für gegebenenfalls substituierte Reste Alkyl, -OR, -SR, -NRR, wobei R und die Substituenten bevorzugt die oben angegebene Bedeutung haben.
- Z: kann außer dem obengenannten Ring gemeinsam mit dem Atom, an welches es gebunden ist und dem Rest 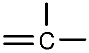 an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Morpholin und N-Methylpiperazin genannt.

Als ganz besonders bevorzugt erfindungsgemäß verwendbare Verbindungen seien Verbindungen der allgemeinen Formeln (II), (III) und (IV) genannt: 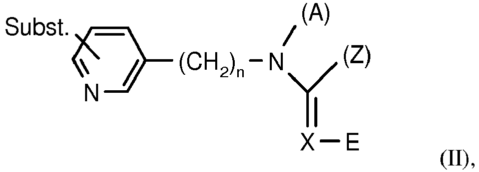 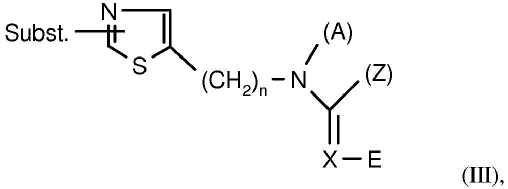 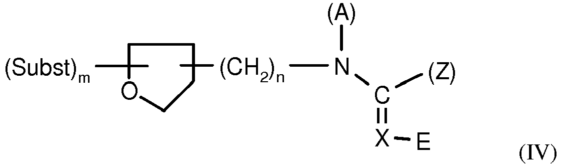 in welchen
- n: für 1 oder 2 steht,
- m: für 0, 1 oder 2 steht,

Subst. für einen der oben aufgeführten Substituenten, insbesonders für Halogen, ganz besonders für Chlor, steht,
A, Z, X und E die oben angegebenen Bedeutungen haben.

Im einzelnen seien folgende Verbindungen genannt:

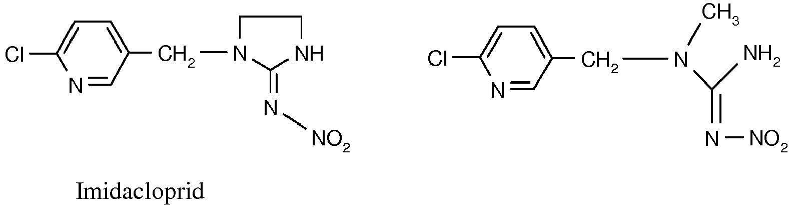

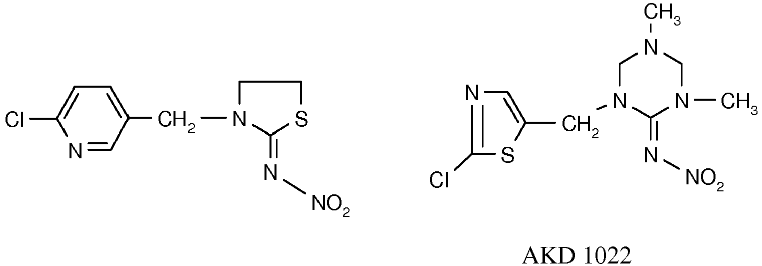

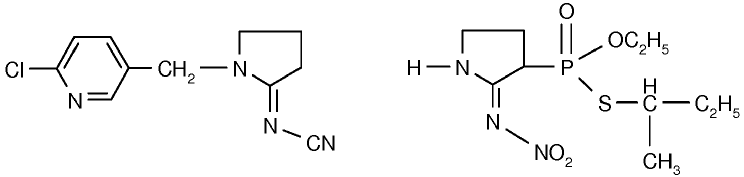

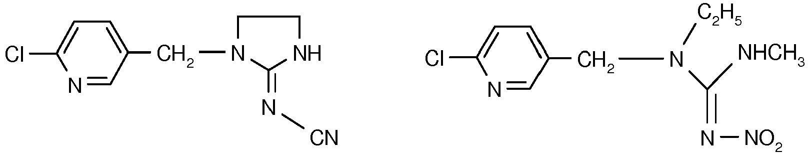

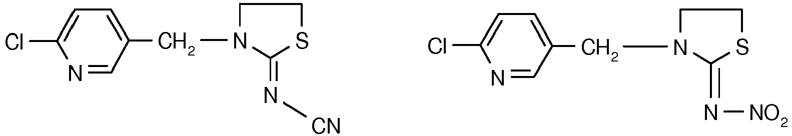

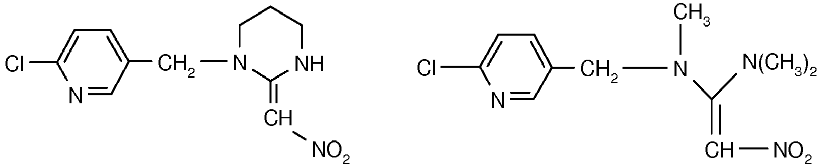

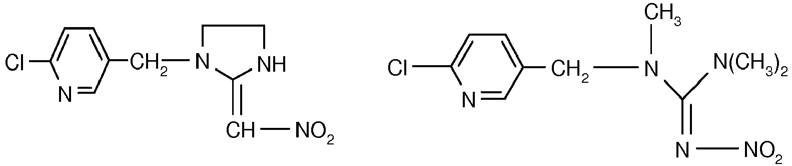

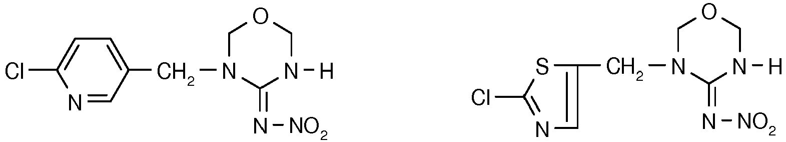

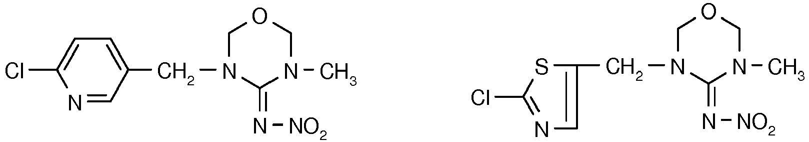

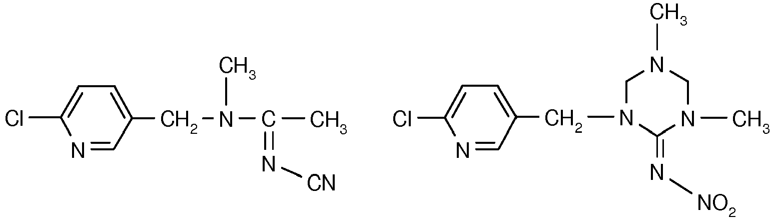

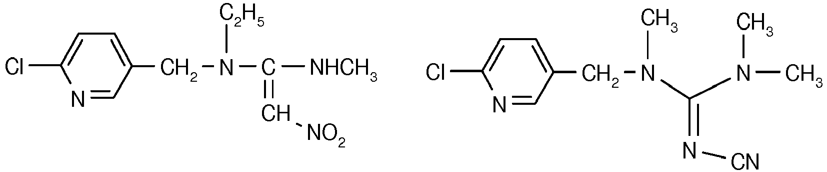

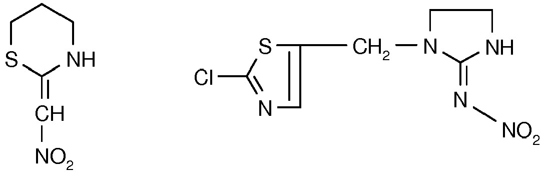

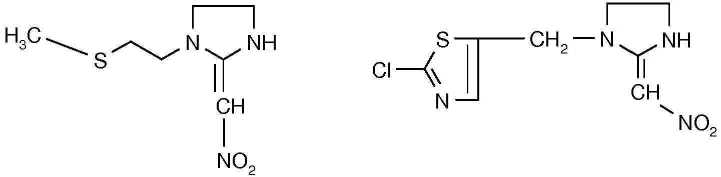

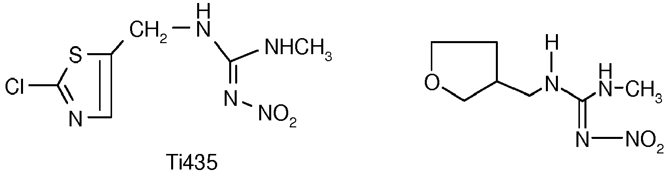

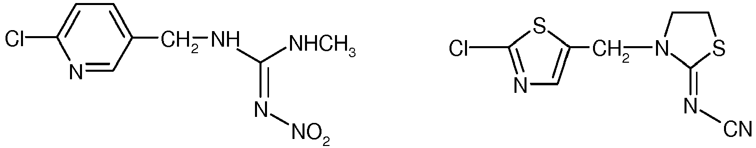

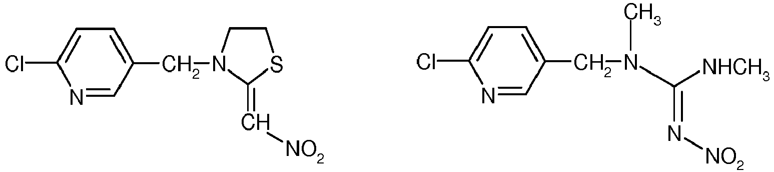

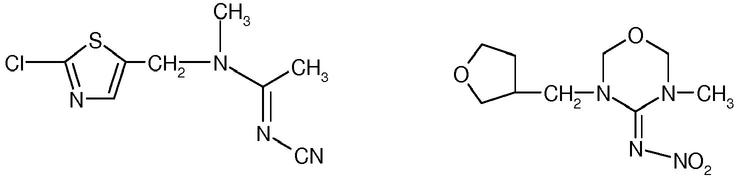

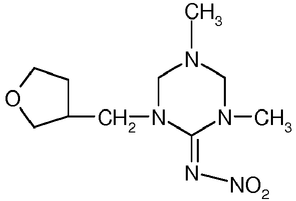

Im einzelnen seien folgende besonders bevorzugte Verbindungen genannt:

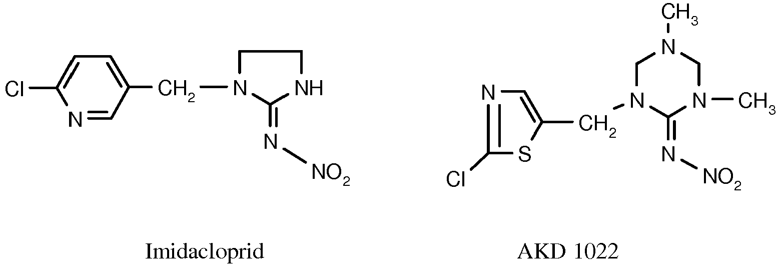

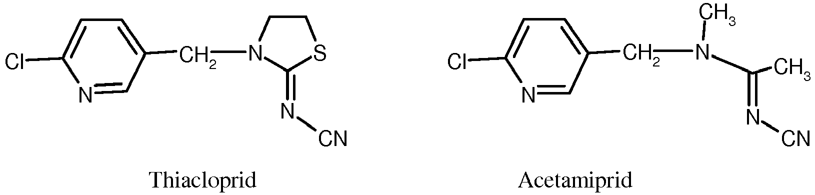

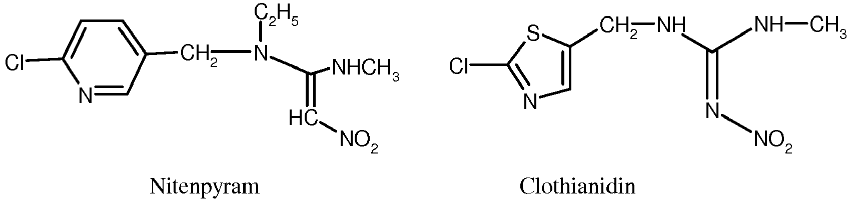

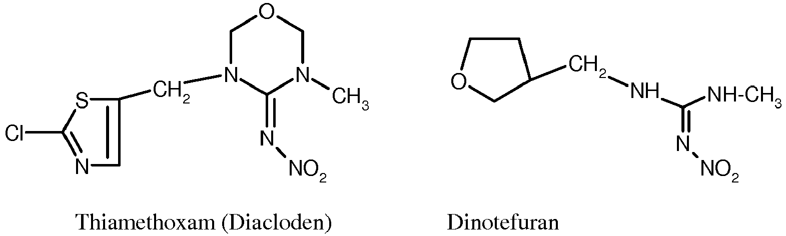

Anwendungsfertige Zubereitungen der erfindungsgemäß verwendbaren Mittel enthalten üblicherweise die Wirkstoffe jeweils in Konzentrationen von 10 ppm bis 30 % m/m; das makrocyclische Lacton wird in Konzentrationen von 0,02 bis 8 m/m eingesetzt; das Neonicotinoid wird in Konzentrationen von 1 bis 20 % m/m, besonders bevorzugt 5-15 % m/m eingesetzt.

Zubereitungen die vor Anwendung verdünnt werden, enthalten die Wirkstoffe in entsprechend höheren Konzentrationen, z. B. 0,5 bis 90 % m/m, vorzugsweise 5 bis 50 % m/m.

Im Allgemeinen hat es sich als vorteilhaft erwiesen, Mengen von etwa 0,01 bis 100 mg Wirkstoff je kg Körpergewicht pro Tag zur Erzielung wirksamer Ergebnisse zu verabreichen, bevorzugte übliche Tagesdosen liegen beim makrocyclischen Lacton im Bereich von 0,05 bis 10 mg/kg, besonders bevorzugt 0,1 bis 8 mg/kg; falls ein Neonicotinoid eingesetzt wird, liegen übliche Tagesdosen bevorzugt im Bereich 1 bis 20 mg/kg, besonders bevorzugt bei 5-10 mg/kg.

Erfindungsgemäß sind pour-on- oder spot-on-Formulierungen. Diese werden in vergleichsweise kleinen Mengen von üblicherweise 0.1 bis 20 ml, vorzugsweise 0.4 bis 10 ml auf einen kleinen Teil der Körperoberfläche des zu behandelnden Tieres aufgebracht.

Solche Formulierungen enthalten das makrocyclische Lacton in Mengen von 0,02 bis 8 % m/m.

Der Gehalt an Neonicotinoid liegt bei 1-20 % m/m, bevorzugt bei 5-15 % m/m.

Als Lösungsmittel für die pour-on oder spot-on Formulierungen eignen sich die oben genannten Lösungsmittel.

Bevorzugt sind hierbei Lösungsmittel, die über sehr gute Lösungseigenschaften für makrocylische Laktone verfügen wie Ethanol, Isopropanol, Propylenglykol, 2-Hexyldecanol, Octyldodecanol, Dibutyladipat, Mittelkettige Triglyceride, Propylenglykoldicaprylat/dicaprat, Propylenglykollaurat, Isopropylmyristat, Isopropylpalmitat, Propylencarbonat, Dipropylenglycolmonomethylether, Diethylenglykolmonoethylether und Ketone.

Bevorzugt sind auch Lösungsmittel, die über gute Spreiteigenschaften verfügen, wie 2-Hexyldecanol, Octyldodecanol, 2-Octyldodecylmyristat, Cetearylisononanoat, Cetearyloctanoat, Cetylethylhexanoat, Coco-caprylat/caprat, Decylcocoat, Decyloleat, Ethyloleat, Isocetylpalmitat, Isopropylmyristat, Isopropylpalmitat, Isostearylisostearat, Octylpalmitat, Octylstearat, Oleylerucat, Mittelkettige Triglyceride, Propylenglykoldicaprylat/dicaprat, Dipropylenglycolmonomethylether, Diethylenglykolmonoethylether, Cetyldimethicon, Dimethicon und Simethicon.

Besonders bevorzugt sind hierbei Lösungsmittel, die über gute Löseeigenschaften für makrocylische Laktone und über gute Spreiteigenschaften verfügen, wie 2-Hexyldecanol, Octyldodecanol, Dibutyladipat, Dipropylenglycolmonomethylether, Diethylenglykolmonoethylether, Mittelkettige Triglyceride, Propylenglykoldicaprylat/dicaprat, Propylenglykollaurat, Isopropylmyristat und Isopropylpalmitat.

Die Lösungsmittel können alleine oder auch in Kombination eingesetzt werden. Ihre Gesamtkonzentration liegt üblicherweise zwischen 10 und 98 % m/m, bevorzugt zwischen 30 und 95% m/m.

Die bevorzugten Spot-on- oder Pour-on-Formulierungen können darüber hinaus übliche pharmazeutische Zusatz- und Hilfsmittel enthalten.

Spot-on- oder Pour-on-Formulierungen können auch als Emulsionskonzentrate formuliert werden. Hierbei sind die Wirkstoffe in erhöhter Konzentration in einem Lösungsmittel zusammen mit einem Dispergierhilfsmittel gelöst. Der Anwender gibt eine bestimmte Menge dieses Konzentrates in Wasser, worauf sich spontan oder nach Umschütteln eine Emulsion bildet. Als Lösungsmittel können die oben genannten Stoffe und als Dispergierhilfsmittel die ebenfalls oben genannten ionogenen und nicht-ionogenen Emulgatoren eingesetzt werden.

In WO 00/30449 werden Formulierungen für makrocyclische Lactone beschrieben, die sich insbesondere zur Spot-on Applikation eignen.

Gemäß einer besonders bevorzugten weiteren Ausführungsform eignet sich die folgende Basis für erfindungsgemäß einsetzbare Formulierungen, insbesondere spot-on Formulierungen:
Als Lösungsmittel A wird Benzylalkohol oder ein gegebenenfalls substituiertes Pyrrolidon, eingesetzt. Gegebenenfalls substituierte Pyrrolidone sind z. B. 2-Pyrrolidon; 1-(C₁-C₁₀)-Pyrrolidon-2 wie 1-Methylpyrrolidon, 1-Ethylpyrrolidon, 1-Octylpyrrolidon, 1-Dodecylpyrrolidon, 1-Isopropylpyrrolidon, 1-(n-, sek.- oder tert.-Butyl)-Pyrrolidon, 1-Hexylpyrrolidon; 1(C₂-C₁₀-Alkenyl)-pyrrolidon-2 wie 1-Vinylpyrrolidon-2; 1-(C₃-C₈-Cycloalkyl)-pyrrolidon-2 wie 1-Cyclohexylpyrrolidon; 1-(3-Hydroxypropyl)-pyrrolidon, 1-(2-Methoxyethyl)-pyrrolidon, 1-(3-Methoxy-proypl)-pyrrolidon, 1-Benzylpyrrolidon. Von diesen besonders bevorzugt ist Benzylalkohol.

Bevorzugt ist die Verwendung des Lösungsmittels A als Gemisch mit einem Co-Lösungsmittel B ausgewählt aus der Gruppe der cyclischen Carbonate und Lactone (bevorzugte Beispiele sind γ-Butyrolacton, Ethylencarbonat und insbesondere Propylencarbonat) wobei Lösungsmittel A einen Anteil von 20 bis 99 % m/m, bevorzugt 40 bis 90 % m/m, besonders bevorzugt 50 bis 90 % m/m und Lösungsmittel B entsprechend 1 bis 80 % m/m, bevorzugt 10 bis 60 % m/m, besonders bevorzugt 10 bis 50 % m/m hat.

In dem Lösungsmittel oder Lösungsmittelgemisch sind der oder die Wirkstoffe sowie gegebenenfalls weitere Hilfs- und Zusatzstoffe gelöst.

Formulierungen dieser Art eignen sich für Moxidectin in Kombination mit einem Neonicotinoid wie Imidacloprid.

Die erfindungsgemäße topische Behandlung der Demodikose ermöglicht eine einfache und bequeme aber dennoch effektive Behandlung der Krankheit. Üblicherweise genügen Applikationen im Abstand von mindestens einer Woche, bevorzugt mindestens zwei Wochen, besonders bevorzugt mindesten drei Wochen, insbesondere alle vier Wochen um gute Behandlungserfolge zu erzielen. Die Behandlung dauert in der Regel 2 bis 4 Monate.

Die folgenden Beispiele für erfindungsgemäß verwendbare Formulierungen erläutern die Erfindung ohne sie in irgendeiner Weise zu begrenzen. Sofern einzelne Formulierungen der folgenden Beispiele ein anderes makrocyclisches Lacton als Moxidectin und/oder kein Neonicotinoid enthalten, handelt es sich nicht um erfindungsgemäße Beispiele sondern um Referenzbeispiele

### Beispiele

### Beispiel 1

### (Referenz)

| | |
|---|---|
| 6 % w/v | Selamectin |
| 6 % v/v | Dipropylenglykolmonomethylether |
| 0,08% w/v | BHT |
| q.s. 100 % v/v | Isopropanol |

### Beispiel 2

### (Referenz)

| | |
|---|---|
| 12 % w/v | Selamectin |
| 12 % v/v | Dipropylenglykolmonomethylether |
| 0,08% w/v | BHT |
| q.s. 100 % v/v | Isopropanol |

### Beispiel 3

### (Referenz)

| | |
|---|---|
| 100 ml | Formulierung enthalten: |
| 6,0 g | Selamectin |
| 5,63 g | Dipropylenglykolmonomethylether |
| 0,08 g | BHT |
| 69,79 g | Isopropanol |

### Beispiel 4

### (Referenz)

| | |
|---|---|
| 100 ml | Formulierung enthalten: |
| 6,0 g | Selamectin |
| 5,63 g | Dipropylenglykolmonomethylether |
| 0,08 g | BHT |
| 69,8 g | Isopropanol |

### Beispiel 5

### (Referenz)

| | |
|---|---|
| 10,0 g | Imidacloprid |
| 0,08 g | Ivermectin |
| 83,1 g | Benzylalkohol |
| 16,5 g | Propylencarbonat |
| 0,10 g | BHT |

### Beispiel 6

### (Referenz)

| | |
|---|---|
| 10,0 g | Imidacloprid |
| 0,20 g | Ivermectin |
| 83,2 g | Benzylalkohol |
| 16,3 g | Propylencarbonat |
| 0,10 g | BHT |

### Beispiel 7

| | |
|---|---|
| 10,0 g | Imidacloprid |
| 2,5 g | Moxidectin |
| 80,7 g | Benzylalkohol |
| 16,5 g | Propylencarbonat |
| 0,10 g | BHT |

### Beispiel 8

| | |
|---|---|
| 10,0 g | Imidacloprid |
| 1,0 g | Moxidectin |
| 82,2 g | Benzylalkohol |
| 16,5 g | Propylencarbonat |
| 0,10 g | BHT |

### Biologisches Beispiel

### Feldstudie: Behandlung der Demodikose bei Hunden

Bei 23 Hunden wurde eine generalisierte Demodikose mit Advocate® spot-on (100 mg Imidacloprid und 25 mg Moxidectin pro ml) behandelt. Das Mittel wurde am Tag 0 und 28 jeweils ein Mal für 4 Wochen appliziert, Hunde bei denen sich am Tag 28 oder 56 noch Demodex-Milben nachweisen ließen wurden ein drittes Mal behandelt, Hunde bei denen am Tag 56 oder 84 noch Demodex-Milben nachgewiesen werden konnten wurden ein viertes Mal behandelt.
87 % der Hunde waren am Ende der Behandlung milbenfrei, bei den übrigen Hunden konnten deutliche Verbesserungen des Krankheitsbildes festgestellt werden.

Als Vergleich wurde in dieser Studie das Produkt Interceptor® (Tabletten enthaltend Milbemycinoxim) getestet. Diese Tabletten wurden täglich oral über 2 bis 4 Monate verabreicht. Die Ergebnisse bei der Demodikose-Behandlung waren denen mit Advocate® vergleichbar, allerdings ist die spot-on Applikation einmal in 4 Wochen von Advocate® deutlich einfacher und bequemer als die tägliche Tablettengabe bei Interceptor®.

## Patentansprüche

1. Verwendung von Moxidectin zur Herstellung von Arzneimitteln in Form einer pour-on oder spot-on Formulierung zur äußerlichen Anwendung zur Behandlung von Demodikose bei Haus- oder Nutztieren, wobei die Formulierung 0,02 bis 8 % (m/m) Moxidectin sowie 1 bis 20 % (m/m) Neonicotinoid enthält und in einer Menge von 0,1 bis 20 ml auf einen kleinen Teil der Körperoberfläche des zu behandelnden Tieres aufgebracht wird, wobei die Wirkstoffe Moxidectin und Neonicotinoid soweit anwendbar auch in Form eines pharmazeutisch annehmbaren Salzes oder Hydrates eingesetzt werden können.

2. Verwendung gemäß Anspruch 1 wobei die Wirkstoffe nicht in Form eines pharmazeutisch annehmbaren Salzes oder Hydrates eingesetzt werden.

3. Verwendung gemäß einem der vorstehenden Ansprüche, wobei die Formulierung als Neonicotinoid Imidacloprid enthält.

4. Verwendung gemäß einem der vorstehenden Ansprüche, wobei das Arzneimittel ein spot-on Arzneimittel ist.

5. Verwendung gemäß einem der vorstehenden Ansprüche zur Behandlung von Demodikose bei Hunden.

## Claims

1. Use of moxidectin for the preparation of medicaments in the form of a pour-on or spot-on formulation for external use for the treatment of demodicosis in domestic animals or livestock, the formulation comprising from 0.02 to 8% (w/w) moxidectin and from 1 to 20% (w/w) neonicotinoid and being applied to a small part of the body surface of the animal to be treated in an amount of from 0.1 to 20 ml, where the active compounds moxidectin and neonicotinoid, in so far as they can be used, can also be employed in the form of a pharmaceutically acceptable salt or hydrate.

2. Use according to Claim 1, where the active compounds are not employed in the form of a pharmaceutically acceptable salt or hydrate.

3. Use according to one of the preceding claims, where the formulation comprises imidacloprid as neonicotinoid.

4. Use according to one of the preceding claims, where the medicament is a spot-on medicament.

5. Use according to one of the preceding claims for the treatment of demodicosis in dogs.

## Revendications

1. Utilisation de moxidectin pour la préparation de médicaments sous la forme d'une formulation d'application en pour-on ou spot-on pour l'application externe pour le traitement de la démodicose chez les animaux domestiques ou de rente, la formulation contenant de 0,02 à 8 % (m/m) de moxidectin ainsi que 1 à 20 % (m/m) de néonicotinoïde et étant appliquée en une quantité de 0,1 à 20 ml sur une petite partie de la surface du corps de l'animal à traiter, les substances actives moxidectine et néonicotinoïde, dans la mesure où elles peuvent être utilisées, pouvant être utilisées également sous la forme d'un sel ou d'un hydrate pharmaceutiquement acceptable.

2. Utilisation selon la revendication 1, dans laquelle les substances actives ne sont pas utilisées sous la forme d'un sel ou d'un hydrate pharmaceutiquement acceptable.

3. Utilisation selon l'une quelconque des revendications précédentes, dans laquelle la formulation contient de l'imidaclopride en tant que néonicotinoïde.

4. Utilisation selon l'une quelconque des revendications précédentes, dans laquelle le médicament est un médicament spot-on.

5. Utilisation selon l'une quelconque des revendications précédentes pour le traitement de la démodicose chez les chiens.
